(19) Europäisches Patentamt
European Patent Office
Office européen des brevets

(11) Publication number: **0 112 739 B1**

# (12) EUROPEAN PATENT SPECIFICATION

(45) Date of publication of patent specification: **22.02.89**

(51) Int. Cl.[4]: **C 07 D 473/08,** C 07 D 221/28, A 61 K 31/02

(21) Application number: **83402220.4**

(22) Date of filing: **18.11.83**

## (54) 3-methoxy-N-methylmorphinane : theophyllinacetic acid (1:2) complex, process for preparing the same and said complex for therapeutical use.

(30) Priority: **19.11.82 ES 517500**

(43) Date of publication of application:
**04.07.84 Bulletin 84/27**

(45) Publication of the grant of the patent:
**22.02.89 Bulletin 89/08**

(84) Designated Contracting States:
**AT BE CH DE FR GB IT LI LU NL SE**

(58) References cited:
**FR-M- 7 348**
**GB-A- 933 141**
**GB-A-1 060 245**

(73) Proprietor: **PRODES S.A.**
**rue Trabajo s/n.**
**San Justo Desvern (Barcelona) (ES)**

(72) Inventor: **Veit, Dagmar Vedrilla**
**Sant Joan Despi**
**Barcelona (ES)**

(74) Representative: **Chameroy, Claude et al**
**c/o Cabinet Malemont 42, avenue du Président Wilson**
**F-75116 Paris (FR)**

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).



Courier Press, Leamington Spa, England.

**Description**

The present invention relates to a new 3-methoxy-N-methylmorphinane: theophyllinacetic acid (1:2) complex, a process for preparing this complex and the complex for therapeutical use.

From GB—A—933141 a N-cyclohexylsulphamate salt of dextrometorphan (= 3-methoxy-N-methylmorphimane) is known. FR—M—3748 discloses inter alia salts of theophyllinyl-7-acetic acid with certain bases but dextrometorphane is not mentioned.

GB—A—1060245 refers to esters of certain alkaloids with theophylline acetic acid.

The complex of this invention contains 2 moles of theophyllinacetic acid per mole of 3-methoxy-N-methylmorphinane and has the following formula:

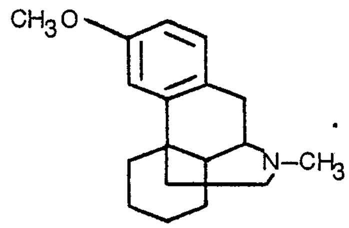

. 2

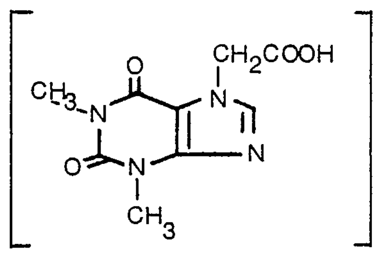

This complex obtained by the process specified in the present invention, has a defined composition formed by 1 mole of 3-methoxy-N-methylmorphinane and 2 moles of theophyllinacetic acid, besides being a stable product with physical properties and spectroscopic data which prove that it deals a complex of a defined structure, completely different from the mixture formed by 1 mole of 3-methoxy-N-methylmorphinane and 2 moles of theophyllinacetic acid.

The process consists in reacting in the presence of a suitable solvent 3-methoxy-N-methylmorphinane with theophyllinacetic acid.

Suitable solvents are polar solvent, as for instance, acetonitrile and the alcohols, preferably using isobutanol.

The used amount of theophyllinacetic acid with respect to 1 mole of 3-methoxy-N-methylmorphinane can oscillate of from 1 to 1.15 mole.

The complex is crystallized by cooling the reaction mass, obtaining unique crystals with a defined smelting point of 187—189°C.

The formation of a chemical complex of a defined structure different from the mixture of the two components if free from any doubt and shown by the hereunder experimental data:

— The product is always formed with a defined proportion (1:2), as shown by the quantitative analysis of the same.

— The product fuses at a fixed temperature (187—189°C) while the mixture of the components makes it in a large margin (100—207°C).

— The infrared spectrum of the complex is different from the cited mixture, concerning intensity as well in the form and position of the most significative bands.

— The product observed under a microscope is formed by a sole type of crystals, while in the mixture of the two components there can be observed the two types of crystals clearly differentiated.

Finally and as most concluding proof that it deals a stable complex of a defined composition, the diffraction spectrum of the product shows diffraction maxima totally different in comparison with the ones of the crystalline species of theophyllinacetic and 3-methoxy-N-methylmorphinane, or of the mixture of both products in the amount (1:2).

The compound according to the present invention is endowed with a *potent antitussive activity.* This activity has been compared with the one of the codeine and the dextromethorphane by the experimental model of Chen, J. Y. P. and col. (1960), consisting in exciting the cavy subjected to an atmosphere of sulfuric acid to cough.

There are used cavy males of a weight of 300—400 g which are treated respectively and as to the lot, with codeine (40 mg/kg; n = 6), dextromethorphane (40 mg/kg; n = 6), theophane (20 mg/kg; n = 5) and theophane (40 mg/kg; n = 6). Each animal acts as his proper control, valuing the number of accesses of cough of the animal before and one hour after the oral administration of the drug.

In Table I below are shown the results obtained by the experience, before and after the administration of the drug as well as the percentage of inhibition of the cough.

TABLE I

| Drug | Animal N° | before | after | Diminution (%) | Average value(%) |
|---|---|---|---|---|---|
| Codeine (40 mg/kg) | 1 | 7 | 2 | 71.4 | 92.2± 12.5 (p<0.001) |
| | 2 | 3 | 0 | 100.0 | |
| | 3 | 11 | 2 | 81.8 | |
| | 4 | 3 | 0 | 100.0 | |
| | 5 | 9 | 0 | 100.0 | |
| | 6 | 3 | 0 | 100.0 | |
| Dextrome-thorphane (40 mg/kg) | 1 | 6 | 4 | 33.3 | 69.7± 27.0 (p < 0.005) |
| | 2 | 10 | 6 | 60.0 | |
| | 3 | 3 | 0 | 100.0 | |
| | 4 | 4 | 1 | 75.0 | |
| | 5 | 2 | 1 | 50.0 | |
| | 6 | 3 | 0 | 100.0 | |
| Theophane * (20 mg/kg) | 1 | 6 | 4 | 33.3 | 44.35±17.5 (p < 0.005) |
| | 2 | 14 | 10 | 28.57 | |
| | 3 | 6 | 4 | 33.3 | |
| | 4 | 3 | 1 | 66.6 | |
| | 5 | 5 | 2 | 60.0 | |
| Theophane * (40 mg/kg) | 1 | 5 | 2 | 60.0 | 70.5±26.6 (p< 0.005) |
| | 2 | 18 | 5 | 72.2 | |
| | 3 | 13 | 9 | 30.7 | |
| | 4 | 3 | 0 | 100.0 | |
| | 5 | 10 | 4 | 60.0 | |
| | 6 | 5 | 0 | 100.0 | |

* Invention compound.

From the results it is deduced that the compound of the present invention with a dosage of 20 and 40 mg/kg/p.o., shows an antitussive activity, significative with regard to its proper control. With a dosage of 40 mg/kg its effect results to be similar to the one produced by dextromethorphane by an equal dosage what, referring to the ponderary index means a greater antitussive activity for theophane.

It has been studied the *bronchodilatory activity "in vitro"* of the compound of the present invention, by using the method of tracheal chain of cavy, described by J. C. Castillo and E. J. J. de Beer in Pharmacol. Expt. Therap. 90,104. It is dealed with the extraction of the trachea of cavy males of a weight of 250—350 g; then the rings are sectioned, always endeavouring that the trachea is bathed by the corresponding perfusion liquid under oxygenation conditions for joining posterioryl the tracheal rings by means of a silk yarn forming the chain giving response to the drugs to be administered. The chain is placed in the bath of organs fixed to the registering arm of a chymograph. The bath is to be at the constant temperature of 37.5°C and using as liquid the Krebs-Hanselett solution.

As bronchoconstrictor drug there is used the histamine at a molar concentration of $1 \times 10^{-5}$.

There has been compared the bronchodilatory activity of theopane (compound according to the invention) with theophylline and the results obtained figure in Table II below.

TABLE II

| Dosage (molar) | Activity (%) | |
|---|---|---|
| | Theophane | Theophylline |
| $1x10^{-5}$ | 0 | 0 |
| $2x10^{-5}$ | - | 0.25 |
| $5x10^{-5}$ | 8.3 | - |
| $1x10^{-4}$ | 54.6 | 10.38 |
| $2x10^{-4}$ | 37.5 | 30 |
| $5x10^{-4}$ | 85.3 | 59.1 |
| $1x10^{-3}$ | 100 | 100 |

The product of the present invention shows a bronchodilatory activity "in vitro" at less concentrations than theophylline, although the time elapsing to obtain the bronchodilatation is larger for theophane than for theophylline. At the molar dosage of $5 \times 10^{-4}$, the time elapsed until obtaining an activity of 50% for theophane is of 280 seconds, while the time for theophylline is only of 180 seconds.

*The acute toxicity* of theophane has been studied orally and i.p. in the mouse and in the rat. There has been determined the $LD_{50}$ and the limits of reliance by the technique of Litchfield and Wilcoxon. Table III below summarizes the values of the obtained $LD_{50}$, as well as of $LD_0$.

TABLE III

| Species | Via | $LD_{50}$ (mg/kg) | $LD_0$ (mg/kg) |
|---|---|---|---|
| RAT | Oral | 429.21 (574.28 / 302.78) | 204.4 |
| | I.P. | 229.94 (262.36 / 201.50) | 156.4 |
| MOUSE | Oral | 359.70 (435.30 / 297.30) | 182.2 |
| | I.P. | 141.81 (162.60 / 113.60) | 102.3 |

*The subacute toxicity* of theophane has been studied after the reiterated oral administration during 1 month in Wistar rats of both sex. The used theophane dosage was 20 mg/kg/day, 60 mg/kg/day and 120 mg/kg/day. A lot of control animals only received excipient. The evolution of mortality, weight rates, diet consumption, hematic, biochemical and urinary parameters, as well as the histophathologic study showed the null toxicity of the theophane product at the dosages as used.

*The fetal and teratogenous toxicity* of theophane studied in Sprague-Dawley rats and fetus of first generation, and using a dosage of 20, 65 and 130 mg/kg/day of the product via oral administration and during the period of organogenesis has shown that the product subject of the present invention does not produce any toxic effects to to gestating females and its breedings and neither induces teratogenous effects.

*The possible mutagenic effect* to theophane has been evaluated according to the method described by Ames, B. N. and cols (1975) using auxotrophic S. typhimurium strains for the histidine. The product has been studied at six different dosages, solved in DMSO, and in the presence and absence of a system of metabolic activation and in front of positive controls. No reversion increase could be observed, consequently the conclusion is that theophane does not have any mutagenic activity in none of the used bacterial strains.

The cardiovascular activity (Technique of Smith, 1961) and the neuropharmacological activity (Irwin Test) of the theophane product have been studied respectively in cavies and rats (males). Increasing concentrations of from 0.5 to 10 mg/kg of theophane don't produce effects on the cardiovascular system. In the Irwin test, dosages of 60 mg/kg of theophane produced similar depressor effects as caused by dextromethorphane at the dosage of 40 mg/kg.

The compound subject of the present invention can be used in human therapy. The daily dosage via oral is comprised of from 40 mg to 120 mg and daily dosage via rectal is comprised of from 80 mg to 160 mg. It can be administered in the form of tablets, recovered tablets, capsules, syrup and suppositories.

Hereunder are some examples of pharmaceutical ways of administering theophane:

*Formula tablets:*

Theophane.................................20 mg

Lactose.......................................40 mg

Starch....................................... 20 mg

Aerosil P—101 ............................63 mg

Magnesium stearate..................1.2 mg

Ac. di. sol.......................................5 mg

*Formula syrup:*

Theophane...............................600 mg

Propylene glycol.........................25 mg

Glycerol......................................20 mg

Tween 20......................................5 mg

Water c.s.p. ..............................150 mg

*Formula suppositories:*

Theophane.................................30 mg

Monolene I.M.-9 ....................... 2.470 g

To facilitate this disclosure, following examples comprise the way of realization only for illustrative purpose and never limiting the investigation.

Example 1

27,140.6 g (100 moles) of 3-methoxy-N-methylmorphinane (MP = 109—11°C) are solved under heat in 100 ml of isobutanol, adding 27,573.1 g (115 moles) of theophyllinacetic acid (MP = 271°C). The obtained

solution is then cooled to 5°C during 24 hours. The formed crystals are filtered and washed with 20 ml of cold isobutanol. There are obtained 17.3 g (40%) of complex whose melting point is 187—190°C.

| | | | |
|---|---|---|---|
| Calc. ($C_{36}H_{45}N_9O_9$): | 57.82%C | 6.06%H | 16.85%N |
| Found: | 57.70%C | 6.15%H | 16.94%N |

The valuation of the components resulted 36.3% of 3-methoxy-N-methylmorphinane and 63.7% of theophyllinacetic acid, corresponding to a molar porportion of 1:2.

The infrared spectrum (KBr) showed among others the following bands: 3520, 3300, 3120, 3000, 2930, 2860, 1700, 1660, 1545, 1460 and 1240 $cm^{-1}$.

UV spectrum λmax. = 273 nm.

$^1$NRMN Spectrum (DMSO $d_6$, δ ppm) 2.5 (s) 3.0 (s); 3.2 (s) 4.1 (s) 4.65 (s) 6.5 and 6.7 (AB system), 7.65 (s).

Diffraction Spectrum of X-rays (recticular space $d_{hkl}$Å):

| | | | | | |
|---|---|---|---|---|---|
| 14.9393 | 12.0341 | 9.7040 | 7.3232 | 6.8699 | 6.7483 |
| 6.1899 | 5.9829 | 5.3250 | 5.1474 | 4.9347 | 4.8350 |
| 4.4701 | 4.2323 | 4.0192 | 3.8759 | 3.7990 | 3.7090 |
| 3.5936 | 3.5441 | 3.5082 | 3.4321 | 3.3392 | 3.3085 |
| 3.2133 | 3.1243 | 3.1243 | 3.0714 | 2.9453 | 2.9057 |
| 2.7921 | 2.7269 | 2.7269 | 2.6378 | 2.5954 | 2.5214 |
| 2.4656 | 2.3523 | 2.2735 | 2.2102 | 2.1542 | 2.1111 |
| 2.0740 | 2.0340 | 1.8869 | 1.8614 | 1.7883 | 1.6708 |

Example 2

1,375 g (5 moles) of 3-methylmorphinane are solved in 10 ml of anhydrous acetonitrile, adding 1,191 g (5 moles) of theophyllinacetic acid. It is cooled to 0°C and filtered. There are obtained 1,121.7 g (60%) of the complex in form of white crystals having a melting point of 187—189°C.

The spectroscopical data and physical constants of the obatined product coincide with those of example 1.

**Claims for the contracting states: BE CH DE FR GB IT LI LU NL SE:**

1. A 3-methoxy-N-methylmorphinane: theophyllinacetic acid (1:2) complex having the formula:

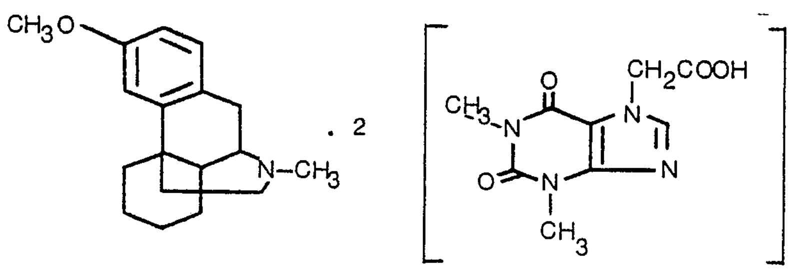

2. A process for preparing the complex according to claim 1, characterized by reacting in the presence of a solvent 3-methoxy-N-methylmorphinane with theophyllinacetic acid.

3. Process according to claim 2, characterized in that the amount of the theophyllinacetic acid oscillates of from 1 to 1.15 mole per mole of 3-methoxy-N-methylmorphinane.

4. Process according to claim 2 or 3, characterized in that the reaction is carried out in a polar solvent.

5. Process according to claim 4, characterized in that the solvent is chosen among acetonitrile and the alcohols.

6. Process according to claim 5, characterized in that the solvent is isobutanol.

7. Process according to any one of the preceding claims, characterized in that the complex is separated from the hot reaction mass by crystallization by cooling to a temperature of 0—5°C and posterior washing with a solvent.

8. The complex according to claim 1 for use as an antitussive and/or brochodilatory agent.

9. Pharmaceutical composition, characterized in that it comprises the complex according to claim 1 with a pharmaceutically acceptable carrier.

**Claims for the contracting state: AT**

1. A process for the obtention of the 3-methoxy-N-methylmorphinane: theophyllinacetic acid (1:2) complex having the formula:

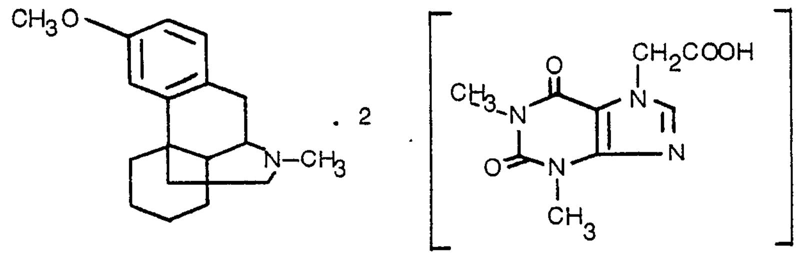

characterized by reacting in the presence of a solvent 3-methoxy-N-methylmorphinane with theophyllinacetic acid.

2. Process according to claim 1, characterized in that the amount of the theophyllinacetic acid oscillates of from 1 to 1.15 mole per mole of 3-methoxy-N-methylmorphinane.

3. Process according to claim 1 or 2, characterized in that the reaction is carried out in a polar solvent.

4. Process according to claim 3, characterized in that the solvent is chosen among acetonitrile and the alcohols.

5. Process according to claim 4, characterized in that the solvent is isobutanol.

6. Process according to any one of the preceding claims, characterized in that the complex is separated from the hot reaction mass by crystallization by cooling to a temperature of 0—5°C and posterior washing with a solvent.

**Patentansprüche für die Vertragsstaaten: BE CH DE FR GB IT LI LU NL SE:**

1. 3-Methoxy-N-methylmorphinan:theophyillinessigsäure-(1:2)-Komplex der Formel:

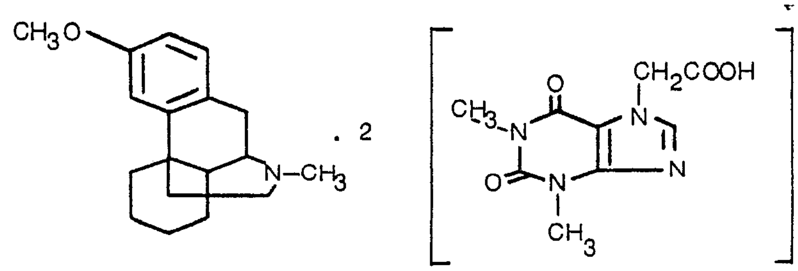

2. Verfahren zur Herstellung des Komplexes nach Anspruch 1, dadurch gekennzeichnet, daß man in Gegenwart eines Lösungmittels 3-Methoxy-N-methylmorphinan mit Theophyllinessigsäure umsetzt.

3. Verfahren nach Anspruch 2, dadurch gekennzeichnet, daß die Menge der Theophyllinessigsäure sich im Bereich von 1 bis 1,15 Mol pro 3-Methoxy-N-methylmorphinan bewegt.

4. Verfahren nach Anspruch 2 oder 3, dadurch gekennzeichnet, daß die Umsetzung in einem polaren Lösungsmittel durchgeführt wird.

5. Verfahren nach Anspruch 4, dadurch gekennzeichnet, daß däs Lösungsmittel unter Acetonitril und den Alkoholen gewählt wird.

6. Verfahren nach Anspruch 5, dadurch gekennzeichnet, daß das Lösungsmittel Isobutanol ist.

7. Verfahren nach mindestens einem der vorangehenden Ansprüche, dadurch gekennzeichnet, daß der Komplex von der heißen Reaktionsmasse durch Kristallisation mittels Kühlen auf eine Temperatur von 0 bis 5°C und anschließendes Waschen mit einem Lösungsmittel abgetrennt wird.

8. Komplex nach Anspruch 1 zur Verwendung als antitussives und/oder bronchodilatorisches Mittel.

9. Arzneimittel, dadurch gekennzeichnet, daß es den Komplex nach Anspruch 1 zusammen mit einem pharmazeutisch annehmbaren Träger umfaßt.

**Patentansprüche für den Vertragsstaat: AT**

1. Verfahren zur Herstellung des 3-Methoxy-N-methylmorphinan:theophyillinessigsäure-(1:2)-Komplex der Formel:

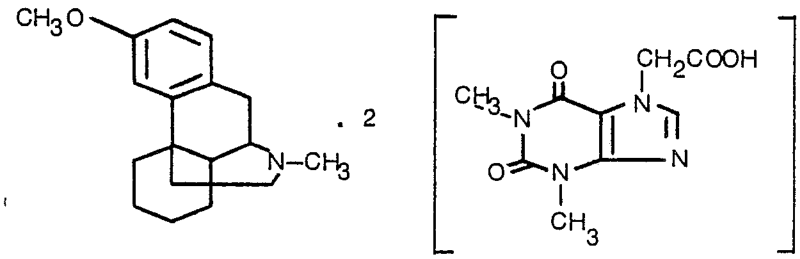

. 2

dadurch gekennzeichnet, daß man in Gegenwart eines Lösungsmittels 3-Methoxy-N-methylmorphinan mit Theophyllinessigsäure umsetzt.

2. Verfahren nach Anspruch 1, dadurch gekennzeichnet, daß die Menge der Theophyllinessigsäure sich zwischen 1 und 1,15 Mol pro Mol 3-Methoxy-N-methylmorphinan bewegt.

3. Verfahren nach Anspruch 1 oder 2, dadurch gekennzeichnet, daß die Umsetzung in einem polaren Lösungsmittel durchgeführt wird.

4. Verfahren nach Anspruch 3, dadurch gekennzeichnet, daß däs Lösungsmittel unter Acetonitril und den Alkoholen gewählt wird.

5. Verfahren nach Anspruch 4, dadurch gekennzeichnet, daß das Lösungmittel Isobutanol ist.

6. Verfahren nach mindestens einem der vorangehenden Ansprüche, dadurch gekennzeichnet, daß der Komplex aus der heißen Reaktionsmasse durch Kristallisation mittels Kühlen auf eine Temperatur von 0—5°C und nachfolgendes Waschen mit einem Lösungsmittel abgetrennt wird.

**Revendications pour les Etats contractants: BE CH DE FR GB IT LI LU NL SE**

1. Complexe méthoxy-3 N-méthylmorphinane: acide théophyllinacétique (1:2), der formule:

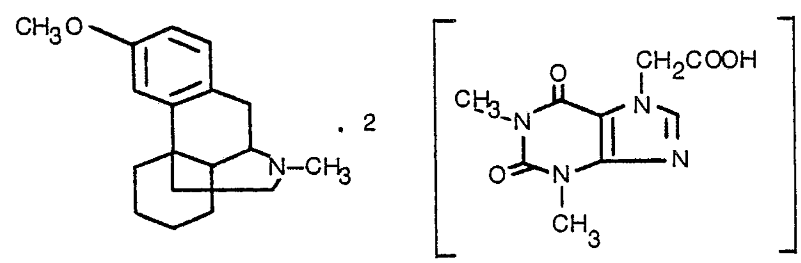

. 2

2. Procédé de préparation du complexe selon la revendication 1, caractérisé par la réaction, en présence d'un solvant, de méthoxy-3 N-méthylmorphinane avec l'acide théophyllinacétique.

3. Procédé selon la revendication 2, caractérisé en ce que la quantité d'acide théophyllinacétique varie de 1 à 1,5 mole par mole de méthoxy-3 N-méthylmorphinane.

4. Procédé selon la revendications 2 ou 3, caractérisé en ce que la réaction est effectuée dans un solvant polaire.

5. Procédé selon la revendication 4, caractérisé en ce que le solvant est choisi parmi l'acétonitrile et les alcools.

6. Procédé selon la revendication 5, caractérisé en ce que le solvant est l'isobutanol.

7. Procédé selon l'une quelconque des revendications précédentes, caractérisé en ce que le complexe est séparé de la masse réactionnelle chaude par cristallisation par refroidissement à une température de 0—5°C puis lavage avec un solvant.

8. Complexe selon la revendication 1, pour utilisation en tant qu'agent antitussif et/ou broncho-dilatatoire.

9. Composition pharmaceutique, caractérisée en ce qu'elle comprend le complexe selon la revendication 1 et un support pharmaceutiquement acceptable.

**Revendications pour l'Etat contractant: AT**

1. Procédé de préparation du complexe méthoxy-3 N-méthylmorphinane: acide théophyllinacétique (1:2), de formule:

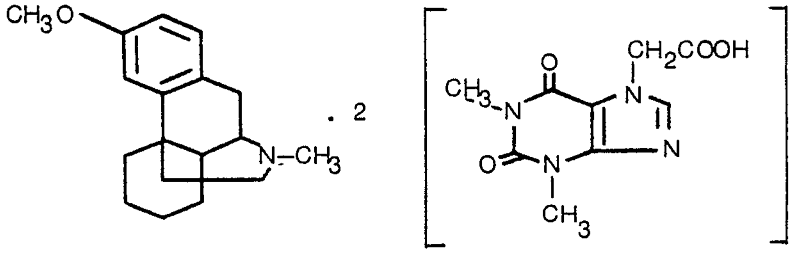

caractérisé par la réaction, en présence d'un solvant, de méthoxy-3 N-méthylmorphinane ave l'acide théophyllinacétique.

2. Procédé selon la revendication 1, caractérisé en ce que la quantité d'acide théophyllinacétique varie de 1 à 1,5 mole par mole de méthoxy-3 N-méthylmorphinane.

3. Procédé selon la revendications 1 ou 2, caractérisé en ce que la réaction est effectuée dans un solvant polaire.

4. Procédé selon la revendication 3, caractérisé en ce que le solvant est choisi parmi l'acétonitrile et les alcools.

5. Procédé selon la revendication 4, caratérisé en ce que le solvant est l'isobutanol.

6. Procédé selon l'une quelconque des revendications précédentes, caractérisé en ce que le complexe est séparé de la masse réactionnelle chaude par cristallisation par refroidissement à une température de 0—5°C puis lavage avec un solvant.